# EUROPEAN PATENT APPLICATION

(11) **EP 3 563 855 A1**
(43) Date of publication of application: **06.11.2019**
(21) Application number: 19175837.4
(22) Date of filing: 07.10.2014
(51) Int. Cl.: A61K 31/7048, A61K 31/12, A61K 45/06, A61P 19/02

(54) **TREATMENT OR PREVENTION OF SYNOVITIS USING ANTI-INFLAMMATORY PHYTONUTRIENTS**

(30) Priority: 14.10.2013 US 201361890626 P
(62) Divisional of application: 14781539.3
(71) Applicant: Société des Produits Nestlé S.A., 1800 Vevey (CH)
(72) Inventor: HORCAJADA, Marie Noëlle, 01170 Echenevex (FR); MEMBREZ, Fanny, 1053 Cugy (CH); OFFORD CAVIN, Elizabeth, 1820 Montreux (CH)
(74) Representative: Chautard, Cécile

(57) **Abstract**

Compositions for treatment or prevention of synovitis are provided. The compositions contain a therapeutically effective amount of at least one of oleuropein, rutin or curcumin and preferably contain at least oleuropin. Methods for treatment or prevention of synovitis are also provided, and the methods include administering such compositions. The compositions can additionally include one or more of quercetin, hydroxytyrosol or vitamin D.

## Description

### BACKGROUND

The present disclosure generally relates to methods and compositions for treatment or prevention of synovitis. More specifically, the present disclosure relates to compositions comprising one or more anti-inflammatory phytonutrients and further relates to methods comprising administering such compositions.

Synovial joints are located where articulating bones contact each other. Synovial joints have a cavity between the bones, and this cavity is lined with a membrane known as the synovial membrane. Synovitis is inflammation of the synovial membrane, and this condition is usually painful, particularly when the joint is moved. The joint usually swells due to synovial fluid collection.

Osteoarthritis (OA) has long been considered a "wear and tear" disease leading to loss of cartilage. OA was previously considered the sole consequence of any process leading to increased pressure on one particular joint or fragility of cartilage matrix. However, the discovery that many soluble mediators, such as cytokines or prostaglandins, can increase the production of matrix metalloproteinases by chondrocytes led to the first steps of an "inflammatory" theory. To date, synovitis is accepted as a critical feature of OA, and some studies are now opening the way to consider synovitis as a driver of the OA process.

Recent experimental data has shown that subchondral bone may have a substantial role in the OA process as a mechanical damper, as well as a source of inflammatory mediators implicated in the OA pain process and in the degradation of the deep layer of cartilage. Thus, initially considered cartilage-driven, OA is a much more complex disease, with inflammatory mediators released by cartilage, bone and synovium. Low-grade inflammation induced by metabolic syndrome, innate immunity, and inflammaging are some of the more recent arguments in favor of the inflammatory theory of OA.

Although there is an increase of individuals suffering from OA, there is still no cure. Current medical therapies remain only palliative, focused on alleviation of symptoms such as pain and inflammation using analgesics (acetaminophen) and non-steroidal anti-inflammatory drugs (NSAID). Furthermore, treatments with these drugs are often associated with side effects such as gastrointestinal or cardiovascular risks, and, as a result, research is still ongoing.

### SUMMARY

The ideal solution for OA would be molecules modifying disease progression in combination with anti-inflammatory and analgesic efficiency and safety. In this context of lack of disease-modifying OA drugs (DMOAD), alternative treatments and OA prevention could come from nutrition. Therefore, the present disclosure provides nutritional solutions to decrease synovitis and thus limit the progression of cartilage degradation during aging.

Accordingly, in a general embodiment, the present disclosure provides a method of treating synovitis. The method includes administering to an individual having synovitis a composition comprising a therapeutically effective amount of a phytonutrient selected from the group consisting of oleuropein, rutin, curcumin and combinations thereof.

In an embodiment, the synovitis is associated with a condition selected from the group consisting of lupus, gout, arthritis and combinations thereof. The condition can be arthritis that is selected from the group consisting of rheumatoid arthritis, osteoarthritis, osteochondritis disease, osteoarthrosis and a combination thereof.

In an embodiment, the phytonutrient comprises oleuropein.

In an embodiment, the composition further comprises an additional bioactive compound selected from the group consisting of quercetin, hydroxytyrosol, vitamin D, and combinations thereof.

In an embodiment, the phytonutrient is isolated from a plant extract.

In another embodiment, a method of preventing synovitis is provided. The method includes administering to an individual at risk thereof a composition comprising a therapeutically effective amount of a phytonutrient selected from the group consisting of oleuropein, rutin, curcumin and combinations thereof.

In an embodiment, the individual is selected from the group consisting of an aging human and an elderly human.

In an embodiment, the phytonutrient is isolated from a plant extract.

In an embodiment, the composition further comprises an additional bioactive compound selected from the group consisting of quercetin, hydroxytyrosol, vitamin D, and combinations thereof.

In an embodiment, the composition is administered daily for at least one month.

In another embodiment, a composition for treating or preventing synovitis is provided. The composition includes a therapeutically effective amount of a phytonutrient selected from the group consisting of oleuropein, rutin, curcumin and combinations thereof.

In an embodiment, the composition is a nutritional composition.

In an embodiment, the nutritional composition comprises a component selected from the group consisting of protein, carbohydrate, fat and combinations thereof.

In another embodiment, a method of treating or preventing articular cartilage degradation subsequent to synovitis is provided. The method includes administering to an individual in need of same a composition comprising a therapeutically effective amount of a phytonutrient selected from the group consisting of oleuropein, rutin, curcumin and combinations thereof.

An advantage of the present disclosure is to treat or prevent synovitis.

A further advantage of the present disclosure is to treat or prevent synovitis using anti-inflammatory nutrients.

Another advantage of the present disclosure is to treat or prevent synovitis more safely than analgesics and NSAIDs.

Still another advantage of the present disclosure is to treat or prevent synovitis without using analgesics or NSAIDs.

An additional advantage of the present disclosure is to treat or prevent synovitis using natural and/or nutritional compounds.

Another advantage of the present disclosure is to treat or prevent synovitis that is associated with lupus, gout, or arthritis such as rheumatoid arthritis or osteoarthritis.

Still another advantage of the present disclosure is to prevent or treat synovitis with tolerable side effects or no side effects.

Yet another advantage of the present disclosure is to treat or prevent synovitis using a nutritional composition at physiological dose.

An additional advantage of the present disclosure is to limit the progression of cartilage degradation during aging.

Another advantage of the present disclosure is to treat or prevent articular cartilage degradation subsequent to synovitis.

Still another advantage of the present disclosure is to treat or prevent synovitis using anti-inflammatory phytonutrients that act synergistically.

Yet another advantage of the present disclosure is to treat or prevent synovitis using at least one of oleuropein, rutin, or curcumin while also providing other polyphenols, such as flavonoids, and minerals and vitamins, at physiological doses.

Additional features and advantages are described herein, and will be apparent from, the following Detailed Description and the Figures.

### BRIEF DESCRIPTION OF THE FIGURES

FIG. 1A shows ADAMTS-5 mRNA expressions relative to GAPDH in absence of ILla which mimics a normal state of the cells. Conditions were statistically compared to CTL-.
FIG. 1B shows ADAMTS-5 mRNA expressions relative to GAPDH in presence of ILla which mimics a disease state of the cells. Conditions were statistically compared to ILla except IL1a which was compared to CTL- (validation of the experiment).
FIG. 2A shows COX-2 mRNA expressions relative to GAPDH in absence of ILla which mimics a normal state of the cells. Conditions were statistically compared to CTL-.
FIG. 2B shows COX-2 mRNA expressions relative to GAPDH in presence of IL1a which mimics a disease state of the cells. Conditions were statistically compared to IL1a except ILla which was compared to CTL- (validation of the experiment).
FIG. 3A shows MMP-13 mRNA expressions relative to GAPDH in absence of IL1a which mimics a normal state of the cells. Conditions were statistically compared to CTL-.
FIG. 3B shows MMP-13 mRNA expressions relative to GAPDH in presence of IL1a which mimics a disease state of the cells. Conditions were statistically compared to ILla except IL1a which was compared to CTL- (validation of the experiment).
FIG. 4 shows histological sections of the knee of guinea pigs (hematoxylin, fast green and safranin-O staining). FIGS. 4A and 4B show Group A (oleuropein); FIGS. 4C and 4D show Group C (rutin); FIGS. 4E and 4F show Group D (rutin and curcumin); and FIGS. 4G and 4H show group B (Control).
FIG. 5 shows total OA score in each group. Group control at week 4 = baseline; group control at week 35; A= group oleuropein (0.025% diet); C= group rutin (0.5% in diet); D: rutin (0.5% in diet) + curcumin (0.25% in diet)
FIG. 6A shows concentration of inflammatory PGE2 biomarker in plasma. Group control at week 4 = baseline; group control at week 35; A= group oleuropein (0.025% diet); C= group rutin (0.5% in diet); D: rutin (0.5% in diet) + curcumin (0.25% in diet)
FIG. 6B shows the correlation between PGE2 levels in plasma at weeks 4 and 35 and corresponding OA score in control group
FIG. 7 shows concentration of Coll2-1NO2 biomarker in plasma linked to collagen 2 breakdown over time. B= group control; A= group oleuropein (0.025% diet); C= group rutin (0.5% in diet); D: rutin (0.5% in diet) + curcumin (0.25% in diet)
FIG. 8A shows concentration of Coll2-1NO2 biomarker in plasma linked to collagen 2 breakdown. Group control at week 4 = baseline; group control at week 35; A= group oleuropein (0.025% diet); C= group rutin (0.5% in diet); D: rutin (0.5% in diet) + curcumin (0.25% in diet)
FIG. 8B shows the correlation between concentration of Coll2-1NO2 biomarker in plasma at weeks 4 and 35 and corresponding OA score in control group
FIG. 9 shows concentration of Fibulin 3-1 biomarker in plasma linked to hypertrophic action of chondrocytes in OA patients. B= group control; A= group oleuropein (0.025% diet); C= group rutin (0.5% in diet); D: rutin (0.5% in diet) + curcumin (0.25% in diet)
FIG. 10A shows concentration of Fibulin 3-1 biomarker in plasma linked to hypertrophic action of chondrocytes in OA patients. Group control at week 4 = baseline; group control at week 35; A= group oleuropein (0.025% diet); C= group rutin (0.5% in diet); D: rutin (0.5% in diet) + curcumin (0.25% in diet)
FIG. 10B shows the correlation between concentration of Fibulin 3-1 biomarker in plasma at weeks 4 and 35 and corresponding OA score in control group.
FIG. 11A shows total synovial score in each group. Group control at week 4 = baseline; group control at week 35; A= group oleuropein (0.025% diet); C= group rutin (0.5% in diet); D: rutin (0.5% in diet) + curcumin (0.25% in diet)
FIG. 11B shows the correlation between total synovial score at weeks 4 and 35 and corresponding OA score in control group.
FIG. 12A shows synovial lining cell hyperplasia in each group. Group control at week 4 = baseline; group control at week 35; A= group oleuropein (0.025% diet); C= group rutin (0.5% in diet); D: rutin (0.5% in diet) + curcumin (0.25% in diet)
FIG. 12B shows the correlation between synovial lining cell hyperplasia at weeks 4 and 35 and corresponding OA score in control group.
FIG. 13A shows the degree of synovial cellular infiltration by perivascular lymphocytes and mononuclear cells in each group. Group control at week 4 = baseline; group control at week 35; A= group oleuropein (0.025% diet); C= group rutin (0.5% in diet); D: rutin (0.5% in diet) + curcumin (0.25% in diet)
FIG. 13B shows the correlation between synovial cellular infiltration at weeks 4 and 35 and corresponding OA score in control group.
FIG. 14 shows collagen synthesis by osteoblasts after 9 days of culture, measured by micrograms of collagen produced per milligram of total protein in response to stimulation with a combination of 1,25(OH)2D3 OLP and HTy.
FIG. 15 shows alkaline phosphatase (ALP) activity after 9 days of culture, indicated by micromolar ALP per milligram of protein per hour in response to with a combination of 1,25(OH)2D3, OLP and HTy.
FIG. 16 shows alkaline phosphatase (ALP) activity of osteoblasts cultured in the presence of 1,25(OH)2D3 after 5, 9, 12 or 15 days of treatment.
FIG. 17A shows alkaline phosphatase (ALP) activity of osteoblasts cultured in the presence of oleuropein after 5, 9, 12 or 15 days of treatment.
FIG. 17B shows alkaline phosphatase (ALP) activity of osteoblasts cultured in the presence of 1,25(OH)2D3 and oleuropein after 5, 9, 12 or 15 days of treatment.
FIG. 18A shows alkaline phosphatase (ALP) activity of osteoblasts cultured in the presence of hydroxytyrosol after 5, 9, 12 or 15 days of treatment.
FIG. 18B shows alkaline phosphatase (ALP) activity of osteoblasts cultured in the presence of 1,25(OH)2D3 and hydroxytyrosol after 5, 9, 12 or 15 days of treatment.
FIG. 19A shows alkaline phosphatase (ALP) activity of osteoblasts cultured in the presence of oleuropein and hydroxytyrosol after 5, 9, 12 or 15 days of treatment.
FIG. 19B shows alkaline phosphatase (ALP) activity of osteoblasts cultured in the presence of 1,25(OH)2D3, oleuropein and hydroxytyrosol after 5, 9, 12 or 15 days of treatment.
FIG. 20 shows the chemical structure of oleuropein.
FIG. 21 shows the chemical structure of rutin.
FIG. 22 shows the chemical structure of curcumin.

### DETAILED DESCRIPTION

All percentages expressed herein are by weight of the total weight of the composition unless expressed otherwise. When reference is made to the pH, values correspond to pH measured at 25 °C with standard equipment. As used in this disclosure and the appended claims, the singular forms "a," "an" and "the" include plural referents unless the context clearly dictates otherwise. As used herein, "about" is understood to refer to numbers in a range of numerals. Moreover, all numerical ranges herein should be understood to include all integers, whole or fractions, within the range. The composition disclosed herein may lack any element that is not specifically disclosed herein. Thus, the disclosure of an embodiment using the term "comprising" includes a disclosure of an embodiment "consisting essentially of" and an embodiment "consisting of' the referenced components. Any embodiment disclosed herein can be combined with any other embodiment disclosed herein.

"Prevention" includes reduction of risk and/or severity of synovitis. The terms "treatment," "treat" and "to alleviate" include both prophylactic or preventive treatment (that prevent and/or slow the development of a targeted pathologic condition or disorder) and curative, therapeutic or disease-modifying treatment, including therapeutic measures that cure, slow down, lessen symptoms of, and/or halt progression of a diagnosed pathologic condition or disorder; and treatment of patients at risk of contracting a disease or suspected to have contracted a disease, as well as patients who are ill or have been diagnosed as suffering from a disease or medical condition. The term does not necessarily imply that a subject is treated until total recovery. The terms "treatment" and "treat" also refer to the maintenance and/or promotion of health in an individual not suffering from a disease but who may be susceptible to the development of an unhealthy condition. The terms "treatment," "treat" and "to alleviate" are also intended to include the potentiation or otherwise enhancement of one or more primary prophylactic or therapeutic measures. The terms "treatment," "treat" and "alleviate" are further intended to include the dietary management of a disease or condition or the dietary management for prophylaxis or prevention a disease or condition. A treatment can be patient-or doctor-related.

As used herein, a "therapeutically effective amount" is an amount that prevents a deficiency, treats a disease or medical condition in an individual or, more generally, reduces symptoms, manages progression of the diseases or provides a nutritional, physiological, or medical benefit to the individual. The therapeutically effective amount that is required to achieve a therapeutic effect will, of course, vary with the particular composition, the route of administration, the age and the condition of the recipient, and the particular disorder or disease being treated.

An "aging" animal has exceeded 50% of the average lifespan for its particular species and/or breed within a species. An animal is considered "elderly" if it has surpassed the first two thirds of the average expected lifespan in its country of origin, preferably if it has surpassed the first three quarters of the average expected lifespan in its country of origin, more preferably if it has surpassed the first four fifths of the average expected lifespan in its country of origin. An "elderly human" means a person with a chronological age of 65 years or older.

"Animal" includes, but is not limited to, mammals, which includes but is not limited to, rodents, aquatic mammals, domestic animals such as dogs and cats, farm animals such as sheep, pigs, cows and horses, and humans. Where "animal," "mammal" or a plural thereof is used, these terms also apply to any animal that is capable of the effect exhibited or intended to be exhibited by the context of the passage. As used herein, the term "patient" is understood to include an animal, especially a mammal, and more especially a human that is receiving or intended to receive treatment, as treatment is herein defined. While the terms "individual" and "patient" are often used herein to refer to a human, the present disclosure is not so limited. Accordingly, the terms "individual" and "patient" refer to any animal, mammal or human, having or at risk for a medical condition that can benefit from the treatment.

"Food product" and "food composition," as used herein, are understood to include any number of optional additional ingredients, including conventional food additives, for example one or more proteins, carbohydrates, fats, acidulants, thickeners, buffers or agents for pH adjustment, chelating agents, colorants, emulsifiers, excipients, flavor agents, minerals, osmotic agents, a pharmaceutically acceptable carrier, preservatives, stabilizers, sugars, sweeteners, texturizers and/or vitamins. The optional ingredients can be added in any suitable amount.

A "kit" means that the components of the kit are physically associated in or with one or more containers and considered a unit for manufacture, distribution, sale, or use. Containers include, but are not limited to, bags, boxes, cartons, bottles, packages of any type or design or material, over-wrap, shrink-wrap, affixed components (e.g., stapled, adhered, or the like), or combinations thereof.

"Phytonutrients" are non-nutritive compounds that are found in many foods. The term "phytonutrients" refers to any compound produced by a plant that imparts one or more health benefits on the user.

Without wishing to be bound by theory, the inventors believe that inflammatory mediators play a pivotal role in the initiation and perpetuation of the OA process. The source of such mediators would be local from joint cells and systemic from other tissues such as adipose tissue released in blood flow and then reaching the joint via the subchondral bone vasculature. These mediators then have a deleterious effect on cartilage, bone and synovium. As an example, joint swelling is one clinical feature of OA attributed to inflammation and reflecting the presence of synovitis due to thickening of the synovium or to effusion.

In this context, the inventors assessed the efficacy of anti-oxidant and anti-inflammatory nutrients on synovitis and cartilage integrity in an aging animal model for OA (Guinea Pig). Curcumin, rutin and oleuropein (and subsequent metabolites) have anti-inflammatory and anti-oxidant properties. The inventors assessed the effect of these compounds on synovitis and subsequent cartilage degradation. Curcumin, rutin and oleuropein significantly improve the joint health by decreasing cartilage degradation and histological OA score, and the group administered oleuropein showed a significant decrease of the synovial modification (synovial score, lining cell hyperplasia and cellular infiltration). Moreover, oleuropein importantly reduced the PGE2 levels found in serum, and in parallel the levels of Coll2-1NO2, a biomarker linked to the oxidative stress in cartilage. This data suggests that oleuropein was able to decrease synovitis (inflammation of the synovium) through an anti-inflammatory pathway (PGE2). In conclusion, oleuropein and rutin significantly slow down the progression of OA lesions in guinea pig developing spontaneously OA. Furthermore, oleuropein significantly decreased PGE2 and COLL2-1NO2 serum levels and reduced synovitis, indicating the potent anti-inflammatory properties of oleuropein. Moreover, synergistic effect of curcumin (with rutin) has been demonstrated through serum levels of fibulin-3.1 (marker of chondrocytes hypertrophy).

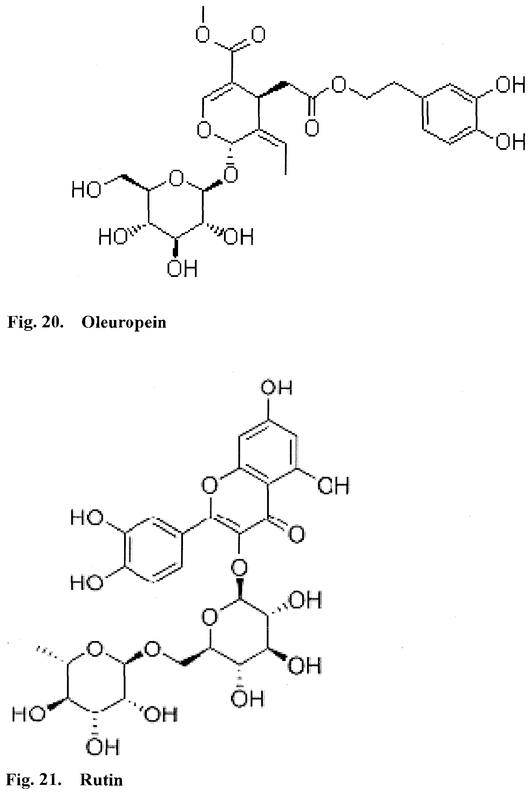

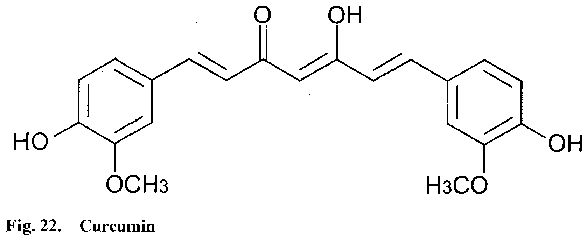

Accordingly, the composition provided by the present disclosure comprises a therapeutically effective amount of an anti-inflammatory phytonutrient selected from the group consisting of oleuropein, rutin, curcumin and a combination thereof. In a preferred embodiment, the composition comprises at least oleuropein. The chemical structures of oleuropein, rutin and curcumin are shown in FIGS. 20-22, respectively.

In an embodiment, synovitis is treated or prevented by administering to an individual in need of same the composition comprising an anti-inflammatory phytonutrient selected from the group consisting of oleuropein, rutin, curcumin and a combination thereof. For example, the composition comprising at least one of oleuropein, rutin or curcumin can be administered to an individual having synovitis to treat the synovitis. The synovitis may be associated with lupus, gout, or arthritis such as rheumatoid arthritis or osteoarthritis. In an embodiment, the composition is administered at least once per day, for a time period of at least 1 month, preferably at least 2 months, and more preferably at least 3 months.

In an embodiment, synovitis is treated or prevented by administering to an aging or elderly individual, such as an elderly human, the composition comprising at least one of oleuropein, rutin or curcumin. However, the present disclosure is not limited to a specific age of the recipient of administration, and the composition can be administered to an individual of any age.

The composition can comprise an additional polyphenol, such as hydroxytyrosol (a metabolite of oleuropein) and/or quercetin (a metabolite of rutin). The composition may comprise an amount of each of oleuropein, rutin, hydroxytyrosol, curcumin and quercetin from 0.01 mg to about 1 g, preferably from 0.1 mg to 1 g, even more preferably from 1 mg to about 1 g of each component per serving of the composition.

The oleuropein, hydroxytyrosol, rutin, curcumin and/or quercetin may be from any suitable source and may be isolated and/or chemically synthesized. In a preferred embodiment, oleuropein, hydroxytyrosol, rutin, curcumin and/or quercetin are obtained from plant sources. For example, oleuropein and hydroxytyrosol may be obtained from olive plants, rutin may be obtained from onions, and curcumin may be obtained from curcuma plants of the ginger family.

The composition may also comprise one or more additional bioactive compounds, such as one or more compounds selected from the group consisting of antioxidants, anti-inflammatory compounds, glycosaminoglycans, prebiotics, fibers, probiotics, fatty acids, minerals, trace elements and vitamins. A "bioactive compound" is any compounds that contributes to the health of an individual or has an effect on the human body, beyond that of meeting basic nutritional need. The one or more additional bioactive compounds may be from a natural source. Thus the compounds may be from extracts of plants, animals, fish, fungi, algae, or microbial fermentation. Minerals are considered to be from a natural source.

In an embodiment, the composition includes one or more additional anti-inflammatory compounds. In an embodiment, the method of treating or preventing synovitis includes administering one or more additional anti-inflammatory compounds sequentially or simultaneously relative to the composition comprising a therapeutically effective amount of at least one of oleuropein, rutin or curcumin. For example, the one or more additional anti-inflammatory compounds may be provided in the same composition as the composition comprising a therapeutically effective amount of at least one of oleuropein, rutin or curcumin. Alternatively, the one or more additional anti-inflammatory compounds may be provided separately from the therapeutically effective amount of at least one of oleuropein, rutin or curcumin, for example in a kit or a series of interacting products. If the composition comprising a therapeutically effective amount of at least one of oleuropein, rutin or curcumin and the one or more additional anti-inflammatory compounds are presented in separate compositions, these compositions may be mixed prior to administration or may be administered simultaneously or sequentially.

The one or more additional anti-inflammatory compounds may be any compound with an anti-inflammatory effect. A non-limiting example of suitable additional anti-inflammatory compounds is omega-3 polyunsaturated fatty acids, which may for example be extracted from fish. Another non-limiting example of suitable additional anti-inflammatory compounds is a plant extract comprising flavonoids, polyphenols, proanthocyanins, and/or lipids such as avocado soybean unsaponifiables and omega-3 polyunsaturated fatty acids. Plant extracts may be from one or more plants selected from the group consisting of devil's claw (*Harpagophytum procumbens*)*,* Rosmarinus officinalis, hyssop, ginger (*Zingiber officinale*)*,* turmeric (*Curcuma longa*)*,* Arnica Montana, willow bark, pomegranate (*Punica granatum*), green tea (*Camellia sinensis*)*,* cat's claw (*Uncaria tometosa* and *Uncaria guianensis*)*,* Indian olibaum (*Boswelia serrata*)*,* and pineapple bromelain (*Ananas comosus*), Black seed (*Nigella sativa*)*,* St. John's wort, hyperforin, Goldenseal, German Chamomile (*Matricaria recutita*) and combinations thereof.

In an embodiment, the composition includes one or more additional antioxidants. In an embodiment, the composition includes an additional compound that may be characterized as both an anti-inflammatory compound and an antioxidant. Non-limiting examples of suitable antioxidants are astaxanthin, carotenoids, coenzyme Q10 (CoQ10), flavonoids, glutathione, Goji (wolfberry), hesperidin, lactowolfberry, lignan, lutein, lycopene, polyphenols, selenium, vitamin A, vitamin C, vitamin E, and zeaxanthin. In preferred embodiments, the antioxidant is from a natural source, such as for example a plant extract.

In an embodiment, the composition includes a glycosaminoglycan. Glycosaminoglycans are large, heavily charged molecules which are present in healthy cartilage. Examples of glycosaminoglycans are glucosamine and chondroitin sulphate. Thus, an embodiment of the composition comprises at least one of glucosamine or chondroitin sulphate.

In some embodiments, the composition includes one or more prebiotics. Non-limiting examples of suitable prebiotics include acacia gum, alpha glucan, arabinogalactans, beta glucan, dextrans, fructooligosaccharides, fucosyllactose, galactooligosaccharides, galactomannans, gentiooligosaccharides, glucooligosaccharides, guar gum, inulin, isomaltooligosaccharides, lactoneotetraose, lactosucrose, lactulose, levan, maltodextrins, milk oligosaccharides, partially hydrolyzed guar gum, pectic oligosaccharides, resistant starches, retrograded starch, sialooligosaccharides, sialyllactose, soyoligosaccharides, sugar alcohols, xylooligosaccharides, their hydrolysates, or combinations thereof.

The composition may include fiber. The fiber may be soluble fiber, insoluble fiber or a combination thereof. Non-limiting examples of soluble fibers include fructooligosaccharides, acacia gum, inulin, agar-agar, an alginate, carob bean, carrageenan, gum arabic, guar gum, karaya gum, pectin or xanthan gum, and the like, and these soluble fibers can be in a hydrolyzed form or a non-hydrolyzed form. A non-limiting example of an insoluble fiber is pea outer fiber.

The composition may include one or more probiotics. Non-limiting examples of suitable probiotics include *Aerococcus, Aspergillus, Bacteroides, Bifidobacterium, Candida, Clostridium, Debaromyces, Enterococcus, Fusobacterium, Lactobacillus, Lactococcus, Leuconostoc, Melissococcus, Micrococcus, Mucor, Oenococcus, Pediococcus, Penicillium, Peptostrepococcus, Pichia, Propionibacterium, Pseudocatenulatum, Rhizopus, Saccharomyces, Staphylococcus, Streptococcus, Torulopsis, Weissella,* non-replicating microorganisms, and combinations thereof.

In some embodiments, the composition includes minerals and trace elements. Non-limiting examples of suitable minerals and trace elements include calcium, magnesium, sodium, potassium, phosphorus, copper, zinc, iron, selenium, chromium and molybdenum, and combinations thereof. Preferably the composition includes calcium and/or magnesium. The calcium may be one or more of, for example, organic and/or inorganic physiologically acceptable compounds, such as calcium inorganic salts (calcium chloride, calcium phosphate, calcium sulphate, calcium oxide, calcium hydroxide and/or calcium carbonate), calcium organic salts (calcium citrate, calcium maleate or mixtures thereof), and/or organic components which comprise calcium, such as powdered skimmed milk or calcium caseinate. The magnesium may be one or more of, for example, organic and/or inorganic physiologically acceptable compounds, such as magnesium inorganic salts (such as magnesium chloride, magnesium phosphate, magnesium sulphate, magnesium oxide, magnesium hydroxide and/or magnesium carbonate), magnesium organic salts (such as magnesium citrate, magnesium maleate or mixtures thereof), and/or organic components which comprise magnesium.

The composition may include vitamins, such as, for example, one or more vitamins selected from vitamin A, vitamin D, vitamin E, vitamin K, vitamin C, folic acid, thiamine, riboflavin, vitamin B6, vitamin B12, niacin, biotin and pantothenic acid. Vitamin D may potentiate the effect of polyphenols such as oleuropein and/or hydroxytyrosol by increasing the effect and/or eliciting the effect earlier in time than in the absence of vitamin D. Vitamin D may be present in any suitable form and preferably in an amount of 800-1200 IU per serving of the composition. Non-limiting examples of suitable forms of vitamin D include cholecalciferol (D3); ergocalciferol (D2); and their biologically active metabolites and precursors such as 1-alpha-hydroxy vitamin D; 25-hydroxy vitamin D; 1,25-dihydroxy vitamin D; and the like.

The composition may be administered by any means suitable for human administration, and in particular for administration in any part of the gastrointestinal tract. Enteral administration, oral administration, and administration through a tube or catheter are all covered by the present disclosure. The composition may also be administered by means selected from oral, rectal, sublingual, sublabial, buccal, topical, and the like.

The composition may be administered in any known form including, for example, tablets, capsules, liquids, chewables, soft gels, sachets, powders, syrups, liquid suspensions, emulsions and solutions in convenient dosage forms. In soft capsules, the active ingredients are preferably dissolved or suspended in suitable liquids, such as fatty oils, paraffin oil or liquid polyethylene glycols. Optionally, stabilizers may be added. If the composition is administered by tube feeding, the nutritional compositions may be used for short term or long term tube feeding.

The composition may be a nutritional composition or a pharmaceutical composition and may be for human or veterinary use. A "nutritional composition" is any composition which is a source of nutrition to an individual. The nutritional composition may be a source of complete nutrition or may be a source of incomplete nutrition. As used herein, "complete nutrition" includes nutritional products and compositions that contain sufficient types and levels of macronutrients (protein, fats and carbohydrates) and micronutrients to be sufficient to be a sole source of nutrition for the animal to which the composition is administered. Patients can receive 100% of their nutritional requirements from such complete nutritional compositions. As used herein, "incomplete nutrition" includes nutritional products or compositions that do not contain sufficient levels of macronutrients (protein, fats and carbohydrates) or micronutrients to be sufficient to be a sole source of nutrition for the animal to which the composition is administered. Partial or incomplete nutritional compositions can be used as a nutritional supplement.

The nutritional compositions provided by the present disclosure may be a medical food. A medical food is a special class of nutritional composition designed to provide dietary management of certain conditions.

In an embodiment, the nutritional composition includes a source of protein. The protein source may be dietary protein including, but not limited to, animal protein (such as milk protein, meat protein or egg protein), vegetable protein (such as soy protein, wheat protein, rice protein, and pea protein), or combinations thereof. In an embodiment, the protein is selected from the group consisting of whey, chicken, corn, caseinate, wheat, flax, soy, carob, pea and combinations thereof.

In an embodiment, the nutritional composition includes a source of carbohydrates. Any suitable carbohydrate may be used in the composition including, but not limited to, starch, sucrose, lactose, glucose, fructose, corn syrup solids, maltodextrin, modified starch, amylose starch, tapioca starch, corn starch, xylitol, sorbitol or combinations thereof.

In an embodiment, the nutritional composition includes a source of fat. The source of fat may include any suitable fat or fat mixture. For example, the fat source may include, but is not limited to, vegetable fat (such as olive oil, corn oil, sunflower oil, high-oleic sunflower, rapeseed oil, canola oil, hazelnut oil, soy oil, palm oil, coconut oil, blackcurrant seed oil, borage oil, lecithins, and the like), animal fats (such as milk fat), or combinations thereof. The source of fat may also be less refined versions of the fats listed above (e.g., olive oil for polyphenol content).

The nutritional composition may also comprise natural or artificial flavors, for example fruit flavors such as banana, orange, peach, pineapple or raspberry or other plant flavors such as vanilla, cocoa, coffee, and the like.

The nutritional compositions may include, besides the main bioactive components, any further bioactive components, and optionally one or more of a protein, carbohydrate and fat source, any number of optional additional food ingredients, including conventional food additives (synthetic or natural), for example one or more acidulants, additional thickeners, buffers or agents for pH adjustment, chelating agents, colorants, emulsifiers, excipient, flavor agent, mineral, osmotic agents, a pharmaceutically acceptable carrier, preservatives, stabilizers, sugar, sweeteners, texturizers, and/or vitamins. The optional ingredients can be added in any suitable amount.

The nutritional composition may be provided in any suitable form. Non-limiting examples of nutritional composition forms in which the composition for use according to the invention may be provided include, solutions, ready-for-consumption compositions (e.g. ready-to-drink compositions or instant drinks), liquid comestibles, soft drinks, juice, sports drinks, milk drinks, milk-shakes, yogurt drinks, soup, and the like. In other embodiments, the nutritional compositions may be provided in the form of a concentrate, a powder, or granules (e.g. effervescent granules), which are diluted with water or other liquid, such as milk or fruit juice, to yield the ready-for-consumption composition. Further nutritional composition forms include baked products, dairy products, desserts, confectionery products, cereal bars, and breakfast cereals. Non-limiting examples of dairy products include milk and milk drinks, yoghurts and other cultured milk products, ice creams and cheeses. Non-limiting examples of baked products include bread, biscuits and cakes.

In an embodiment, the nutritional composition may be provided in a form designed as an animal food, in particular for a dog or a cat, in a wet form, semi-wet form or dry form, in particular in the form of biscuits.

In an embodiment, the composition is a pharmaceutical composition. A pharmaceutical composition is a composition, other than a nutritional composition, used on or in the body to prevent, diagnose, alleviate, treat or cure a disease in humans or animals. The pharmaceutical composition may be for use by a human or alternatively may be a veterinary composition, for example suitable for a dog, a cat, or a horse, in particular a thoroughbred horse.

The pharmaceutical composition can be suitable for oral, parenteral or intravenous administration. When the pharmaceutical composition comprises at least one pharmaceutically or physiologically acceptable excipient, the excipient is preferably appropriate for administration of the composition by the oral route or suitable for administration of the composition by the parenteral route. The pharmaceutical composition can have a solid or liquid form. For oral administration, a solid pharmaceutical composition in the form of tablets, capsules or gelatin capsules is preferred. In liquid form, the pharmaceutical composition can be an aqueous or non-aqueous suspension, or also a water-in-oil or oil-in-water emulsion.

Solid pharmaceutical forms may comprise as vehicles, adjuvants or excipients one or more of a diluent, a flavor, a solubilizing agent, a lubricant, a suspension agent, a binder, a disintegrating agent or an encapsulating agent. Non-limiting examples of such compounds include magnesium carbonate, magnesium stearate, talc, lactose, pectin, dextrin, starch, gelatin, cellulosic materials, cocoa butter, and the like. The pharmaceutical compositions in liquid form may comprise water, optionally as a mixture with propylene glycol or polyethylene glycol, and also may comprise one or more coloring agents, flavors, stabilizers and thickening agents.

In an embodiment, the present disclosure provides a method of manufacturing a nutritional composition for treating or preventing synovitis, and the method comprises mixing oleuropein and/or hydroxytyrosol with one or more other ingredients to form the nutritional composition. The one or more other ingredients can comprise curcumin and/or quercetin.

### EXAMPLES

The following non-limiting examples present scientific data developing and supporting the concept of treatment or prevention of synovitis using a phytonutrient selected from the group consisting of oleuropein, rutin, curcumin and combinations thereof.

### Example 1

An in vitro study of bovine primary chondrocytes in alginate beads was performed. Substantially purified oleuropein (>98%) was purchased from Extrasynthese (Genay, France). Substantially purified hydroxytyrosol (>98%) was purchased from Extrasynthese (Genay, France). Substantially purified quercetin (>97%) was purchased from HWI Analytik GmbH (Ruelzheim, Germany). Substantially purified curcumin (>94%) was purchased from Sigma-Aldrich (Buchs, Switzerland).

The foot of a young cow was washed to remove soil. The skin was removed from the foot. The articulation was opened transversally. Intra articular ligaments were transected. The opened articulation was washed with phosphate buffered saline. With a scalpel blade, full thickness slices of cartilage were dissected out and collected in DMEM high glucose with 10mM Hepes and 1% Penicillin-Streptomycin. Slices of cartilage were washed in DMEM high glucose with 10mM Hepes and 1% Penicillin-Streptomycin.

Cartilage was then digested successively by 0.5 mg/ml of hyaluronidase during 30 minutes, 1.0 mg/ml of Pronase E during 60 minutes and 1.0 mg/ml of collagenase overnight under slow agitation. After the enzymatic incubation, the cell suspension was filtered through a 70 µm cell strainer to separate cell cluster and remove tissue debris. The cell suspension was centrifuged to pellet the cells. The media was discarded and the cell pellet was washed by centrifugation and re-suspended in a 0.9% sodium chloride solution containing 10 mM HEPES.

Cells were suspended in a solution of 0.9% NaCl, 10 mM HEPES containing 1.2% alginate to obtain a density of 4.3x10⁶ cells per ml. The suspension was passed through a needle in a drop-wise fashion into a 10²mM CaCl₂ solution. After 10 min, the beads were polymerized and were washed with 0.9% NaCl, 10 mM HEPES. 10 beads were deposited in each well of a 24 wells culture plate with 1 ml of complete medium (DMEM high glucose with 2mM L-glutamine, 0.5 mg/ml ascorbic acid, 0.2 mg/ml L-proline and 10% fetal calf serum). Cells were maintained in the medium for 2 days and then treatments of 1.5 µM of oleuropein, 1.5 µM of hydroxytyrosol, 1.5 µM of quercetin, 1.5 µM of curcumin, 1.5 µM of oleuropein + 1.5 µM of quercetin, 1.5 µM of oleuropein + 1.5 µM of curcumin, 1.5 µM of hydroxytyrosol + 1.5 µM of quercetin, 1.5 µM of hydroxytyrosol + 1.5 µM of curcumin, 1.5µM of quercetin + 1.5µM of curcumin, 1.5 µM of oleuropein + 1.5µM of quercetin + 1.5µM of curcumin, 1.5 µM of hydroxytyrosol + 1.5 µM of quercetin + 1.5 µM of curcumin in the presence or in the absence of interleukin-1 alpha (10ng/ml). Treatments were dissolved in dimethylslfoxide (DMSO). The final concentration of DMSO was 0.1%. The cells were exposed to treatments for 3 days.

At the end of the treatments, the beads were washed and re-suspended in 0.1 M sodium citrate + 0.15 M sodium chloride solution. The suspension was centrifuged, and the pellet containing cells were collected for total RNA extraction.

Total RNA was isolated from a pool of 40 beads and isolated using RNeasy kit from Qiagen. cDNA was obtained by reverse transcription of RNA with the Primescript 1st strand cDNA Synthesis kit from Takara. mRNA expression of ADAMTS-5, COX-2, MMP-13 and GAPDH were quantified in real-time PCR (SYBR Green). Primers (from Bos Taurus) are listed below in Table 1:

**Table 1. Primers**

| Gene | Forward primer | Reverse primer |
|---|---|---|
| GAPDH | AAG GGC ATT CTA GGC TAC ACT GA (SEQ ID NO. 1) | AGA GTG AGT GTC GCT GTT GAA GTC (SEQ ID NO. 2) |
| ADAMTS-5 | CAC CTC AGC CAC CAT CAC AG (SEQ ID NO. 3) | AGT ACT CTG GCC CGA AGG TC (SEQ ID NO. 4) |
| COX-2 | CAC CCA TCA ATT TTT CAA GAC AGA (SEQ ID NO. 5) | CCA CCC CAT GGT TCT TTC C (SEQ ID NO. 6) |
| MMP-13 | GCA GAG AGC TAC CTG AAA TCA TAC TAC T (SEQ ID NO. 7) | AAT CAC AGA GCT TGC TGC AGT TT (SEQ ID NO. 8) |

In disease conditions, additions of OLP, HTY, QRC or CUR can counteract IL-1 alpha stimulated chondrocytes and decrease expression of MMP-13. Combinations of OLP, HTY, QRC or CUR by three have a synergistic effect.

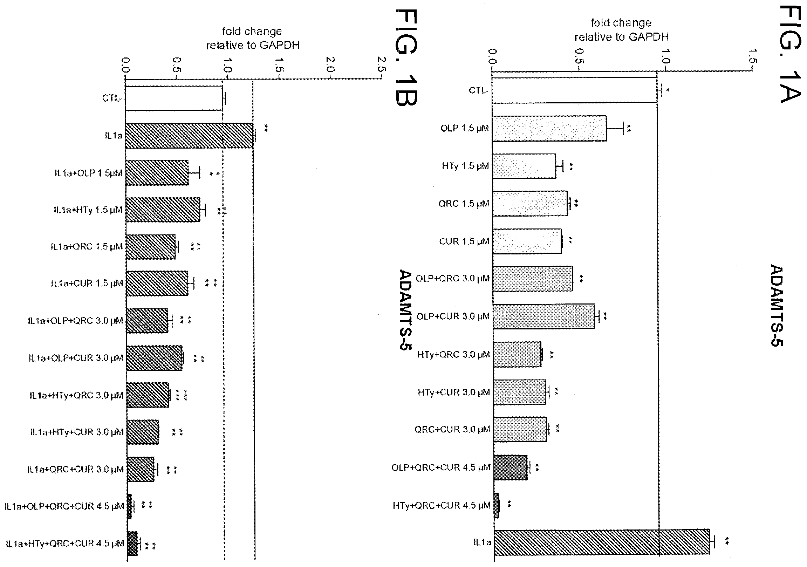

The results were as follows. FIGS. 1-3 show mRNA expression of ADAMTS-5, COX-2 and MMP-13 normalized by GAPDH mRNA expression after 3 days of culture. Data were expressed as fold change in gene expression compared to negative control (cells cultured in complete medium). Results were calculated according to the 2^{-ΔΔCT} method.
- CTL-:: negative control
- OLP:: oleuropein
- HTY:: hydroxytyrosol
- QRC:: quercetin
- CUR:: curcumin
- IL1a:: interleukin-1 alpha 10 ng/ml

Data was expressed as median ± S.E.Median (2 experiments of 3 replicates each). Statistics were done by Kruskal Wallis test followed by Mann Whitney U test (2 tailed) *p<0.05; **<0.01; ***p<0.001 vs negative control (CTL-) or interleukin-1 alpha (IL1a).

FIG. 1A shows ADAMTS-5 mRNA expressions relative to GAPDH in absence of ILla which mimics a normal state of the cells. Conditions were statistically compared to CTL-. FIG. 1B shows ADAMTS-5 mRNA expressions relative to GAPDH in presence of ILla which mimics a disease state of the cells. Conditions were statistically compared to ILla except IL1a which was compared to CTL- (validation of the experiment).

In normal conditions, additions of OLP, HTY, QRC or CUR decrease expression of ADAMTS-5 (marker of cartilage destruction). Combinations of these different polyphenols have a synergistic effect.

In disease conditions, additions of OLP, HTY, QRC or CUR can counteract IL-1 alpha stimulated chondrocytes and decrease expression of ADAMTS-5. Combinations of these different polyphenols have a synergistic effect.

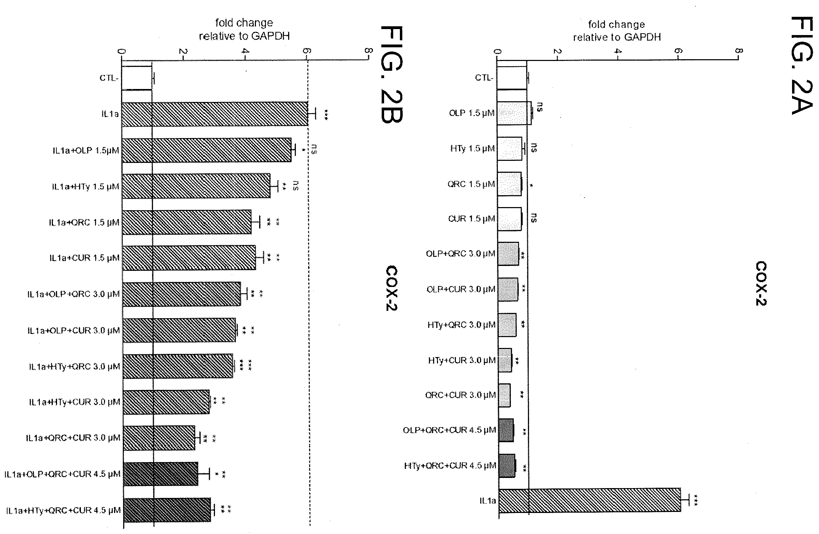

FIG. 2A shows COX-2 mRNA expressions relative to GAPDH in absence of ILla which mimics a normal state of the cells. Conditions were statistically compared to CTL-.

FIG. 2B shows COX-2 mRNA expressions relative to GAPDH in presence of IL1a which mimics a disease state of the cells. Conditions were statistically compared to IL1a except ILla which was compared to CTL- (validation of the experiment).

In normal conditions, addition of QRC can reduce expression of COX-2 (marker of inflammatory status). Combinations of OLP, HTY, QRC or CUR by two or three have a synergistic effect. In disease conditions, additions QRC or CUR can counteract IL-1 alpha stimulated cells and decrease expression of COX-2. Combinations of OLP, HTY, QRC or CUR by two or three have a synergistic effect.

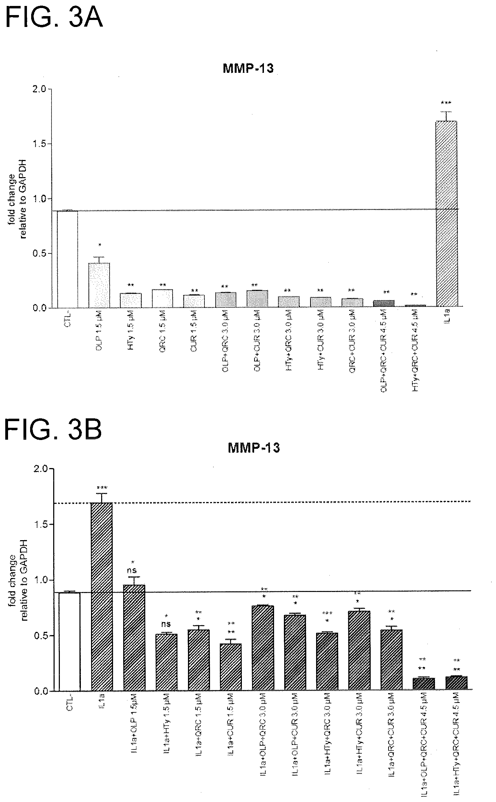

FIG. 3A shows MMP-13 mRNA expressions relative to GAPDH in absence of IL1a which mimics a normal state of the cells. Conditions were statistically compared to CTL-.

FIG. 3B shows MMP-13 mRNA expressions relative to GAPDH in presence of IL1a which mimics a disease state of the cells. Conditions were statistically compared to ILla except ILla which was compared to CTL- (validation of the experiment).

In normal conditions, additions of OLP, HTY, QRC or CUR alone or in combination decrease expression of MMP-13 (involved in the breakdown of extracellular matrix, linked to articular cartilage turnover in OA).

### Example 2

An *in vivo* study of spontaneous development of osteoarthritis in Dunkin-Hartley guinea pigs was performed. Sixty-five male Dunkin-Hartley guinea-pigs aged 3 weeks were obtained from Charles River Laboratories (Paris). Animals were housed 5 per cage in solid bottom cages and fed with standard guinea-pig diet and water ad libitum. All animals were allowed one week for acclimatization to housing conditions prior to phytotnutrients administration. PVC pipes were added to the cages to improve housing conditions and minimize stress.

After one week, the 65 male 4-weeks old Dunkin-Hartley guinea-pigs were randomized in 3 treated groups, receiving a diet containing 0.025% of oleuropein, or 0.5% of rutin or 0.5% of rutin with 0.25% of curcumin during 31-weeks and a control group receiving standard chow diet. Substantially purified oleuropein (>90%) was purchased from Extrasynthese (Genay, France). Substantially purified rutin (>94%) was purchased from Sigma-Aldrich (Buchs, Switzerland). Substantially purified curcumin (>70%) was purchased from Sigma-Aldrich (Buchs, Switzerland).
Group A (n=15) received a daily dose of oleuropein during 31 weeks.
Group B (n=15) received a standard diet during 31 weeks (Control).
Group C (n=15) received a daily dose of rutin during 31 weeks.
Group D (n=15) received a daily dose of rutin and curcumin during 31 weeks.
Group E (n=5, Reference group) were euthanized at inclusion and did not receive any intervention.

Oleuropein, rutin and curcumin were integrated in the diet. Animals were weighed each week and identification was made by microchip. Food intake was controlled at W9, W15, W21, W26 and W34 by weighing daily quantities of food added and remaining.

6h fasting blood was obtained in the morning, under ketamine/xylazine tranquilization, at the superficial veins at the ears, each 6 weeks: W4 (=T0), W10, W16, W22, W28. At W35, blood was collected by intracardiac puncture just before euthanasia, under general anesthesia.

After weighing, animal euthanasia was performed after intracardiac blood puncture under sodium pentobarbital 100mg/kg. Main vital organs were observed to detect anomalies (heart, digestive and urinary tracts, adrenal glands). Liver and adrenal glands were weighed. The right knee joint from each animal was fixed for 24h in 4% buffered paraformaldehyde, followed by decalcification in HCl acid (DC2, Labonord, Belgium) for 4h at 4 °C before paraffin embedding.

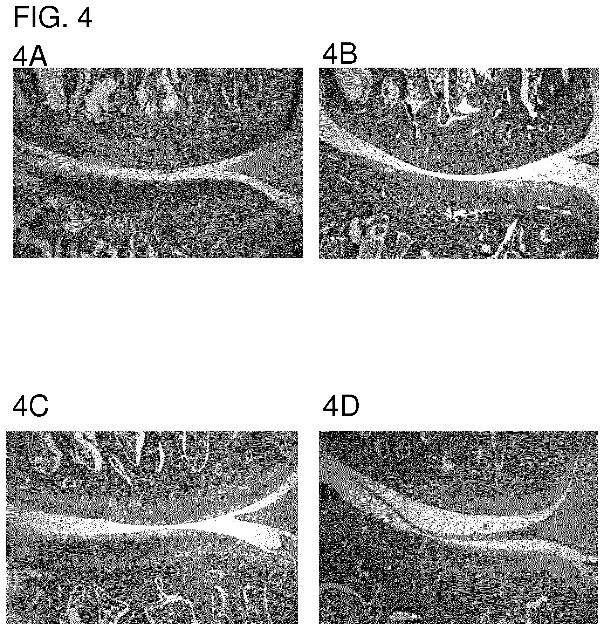

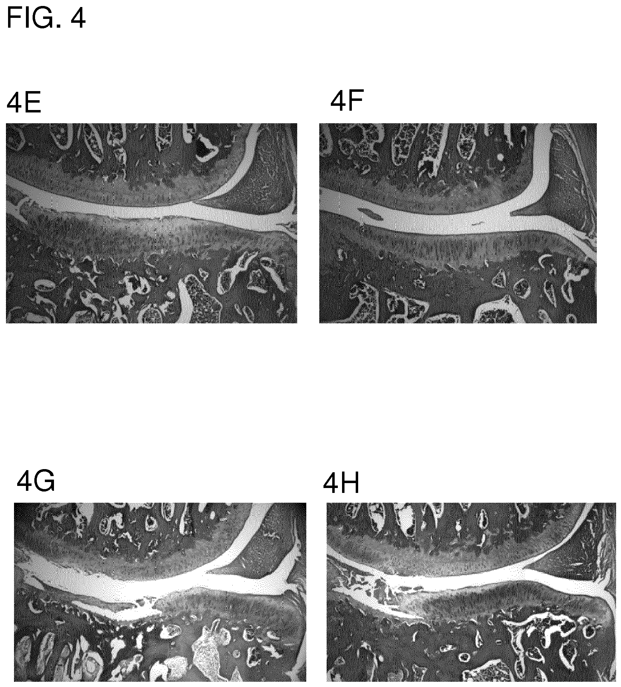

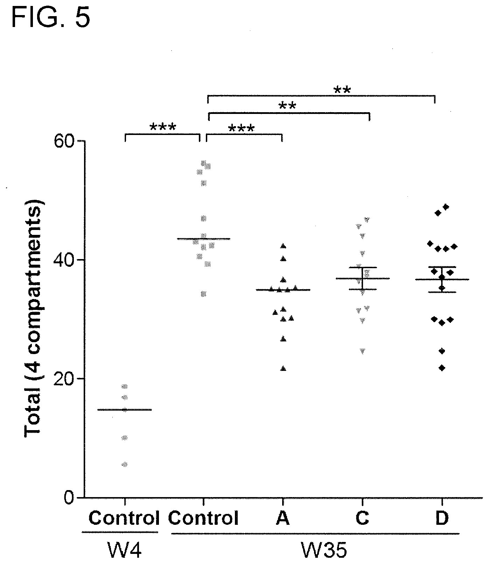

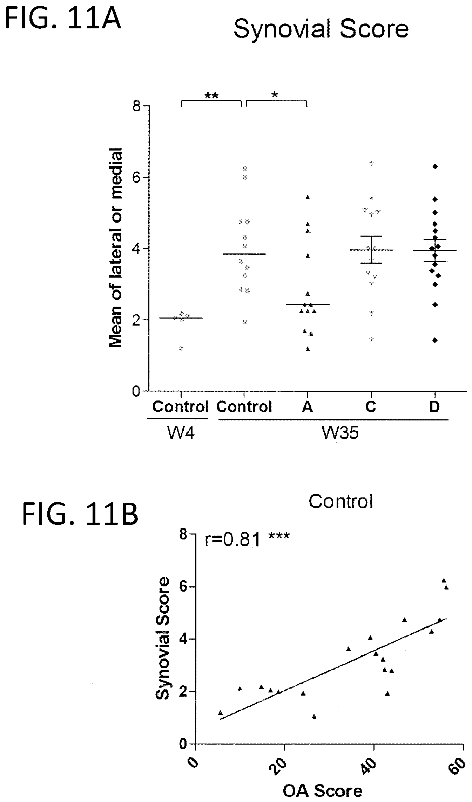

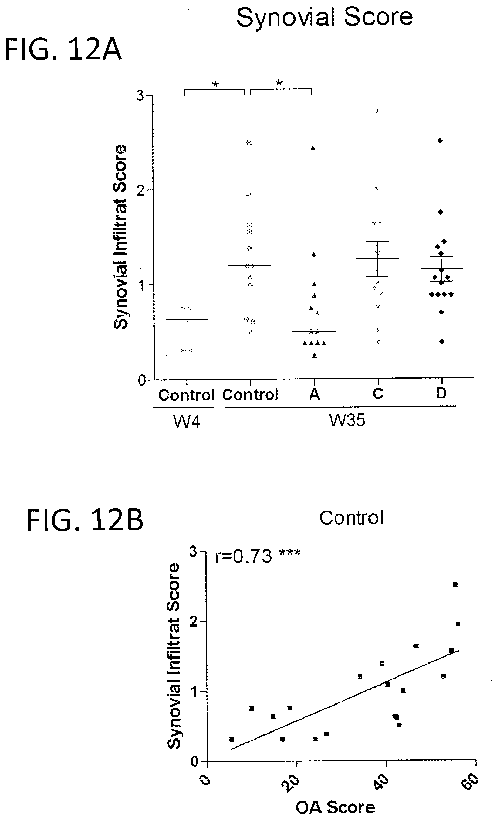

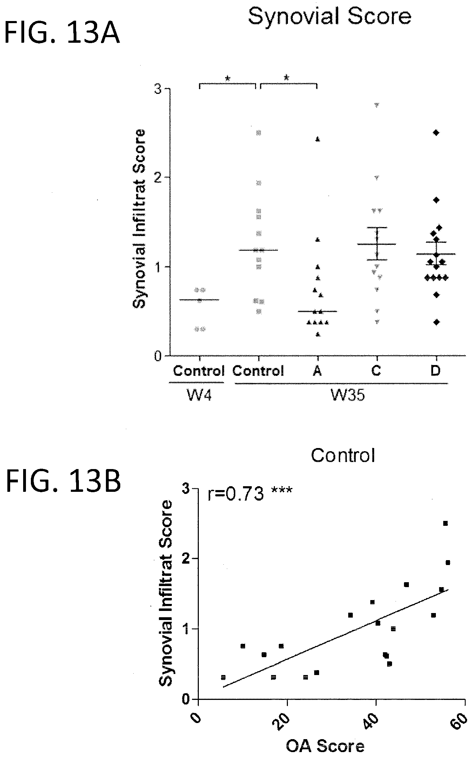

Paraffin embedded right knees were cut with a microtome (Leica) in 6 µm sections, in the central area not covered by meniscus, following the Cushin plane, as recommended by OARSI. Three sections at 200 µm interval were stained with hematoxylin, fast green and safranin-O, and one supplementary central section was stained with toluidine blue. Each compartment of the section (tibial median, tibial lateral, femoral median and femoral lateral) was scored by two blinded trained experts following OARSI recommendation and to assess the global cartilage score, each compartment score was added. Results are shown in Figures 4 and 5. Lateral and medial synovial membranes were scored by 2 blinded trained experts following OARSI recommendation (Table 2) and the mean of lateral and membrane was calculated to assess the global synovial score. Results are shown in Figures 11 to 13.

Oleuropein, rutin and rutin+curcumin groups showed a significant decrease of the OA score (p<0.01) compared to the control group as shown in figure 5.

Significantly, the oleuropein group showed a significant decrease of the synovial score (p<0.05) compared to the control group, as shown in FIGS. 11A, 12A and 13A. The synovial score was positively correlated with the OA score in the control group (Figures 11B, 12B and 13B)

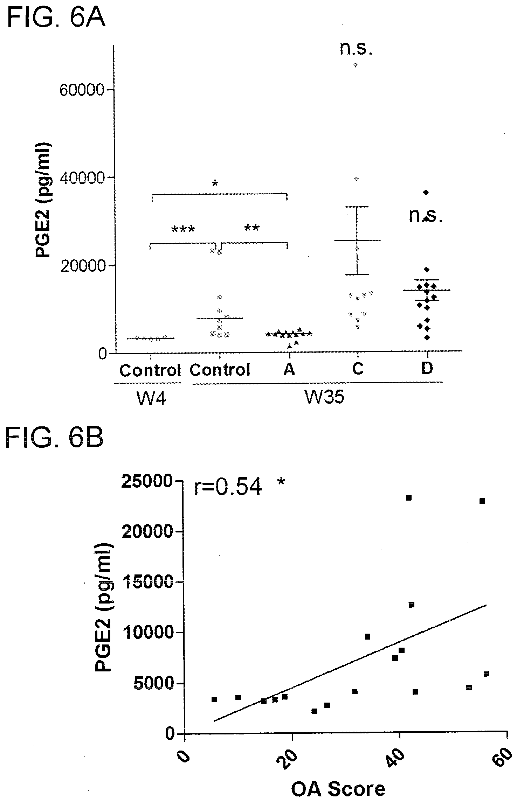

PGE2 was measured in guinea pig serum using a competitive ELISA kit (Arbor Assays, USA). The results are shown in FIG. 6. Oleuropein treatment can decrease PGE2 levels in plasma after 35 weeks of treatments. However, rutin and curcumin had only non-significant effect on PGE2 levels, indicating that their effect was mainly on the breakdown of cartilage.

PGE2 level in plasma can be positively correlated to OA score. This confirms that PGE2 is a relevant biomarker to OA onset and progression in guinea pig model and to evaluate efficacy of treatments.

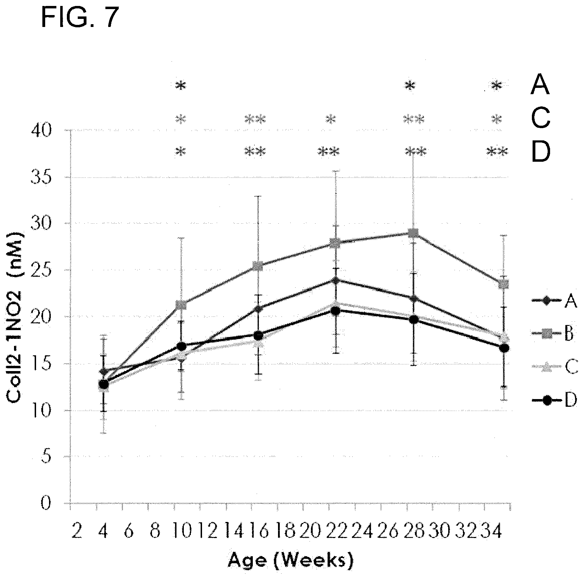

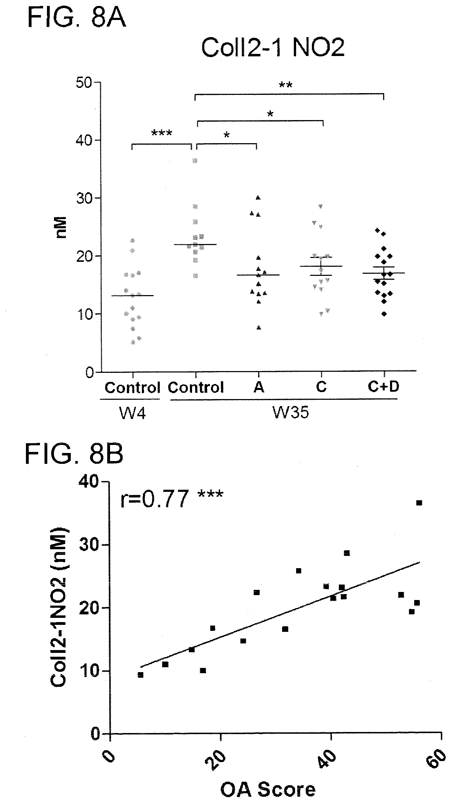

The nitrosylated epitope, Coll2-1NO2, localized to the helical domain of type II collagen was quantified in guinea-pig sera by competitive ELISA in triplicate with polyclonal rabbit antisera (D37, Artialis, Belgium). The Coll2-1NO2 immunoassay quantified with a high specificity and affinity the nitrated amino acids sequence. The results are shown in FIGS. 7 and 8.

Oleuropein, rutin, and rutin+curcumin treatments can decrease collagen breakdown measured by levels of Coll2-1NO2 in plasma. The amount of Coll2-1NO2 is positively correlated to the OA score. This confirms that Coll2-1NO2 is a relevant biomarker to up OA onset and progression in guinea pig model and to evaluate efficacy of treatments.

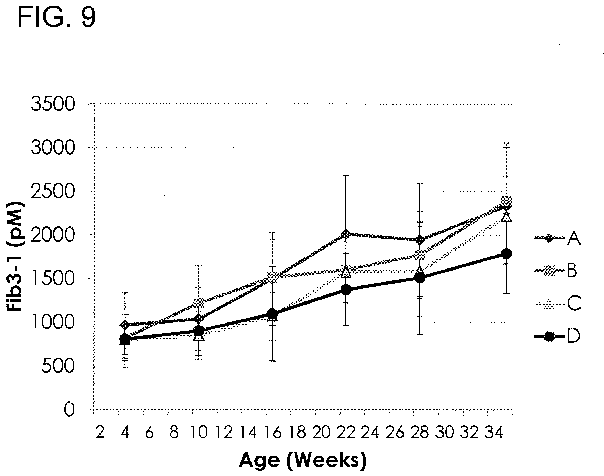

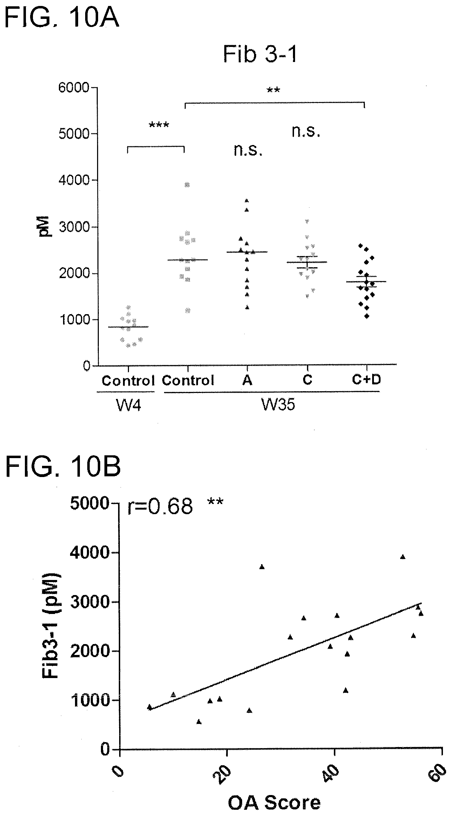

Fib3-1 is fragments of Fibulin-3 increased in sera of OA patients. Fib3-1 was quantified in guinea pig sera by competitive ELISA in triplicate with polyclonal rabbit antisera (AS88, Artialis, Belgium). The results are shown in FIGS. 9 and 10.

Rutin treatment and rutin+curcumin treatment can decrease Fibulin 3-1 levels in plasma. Fibulin 3-1 level in plasma can be positively correlated to OA score. This confirms that Fib 3-1 is a relevant biomarker to OA onset and progression in guinea pig model and to evaluate efficacy of treatments.

The intra- and inter-assays CVs were lower than 10% and the dilution curves were parallel to the standard curve for all biomarker assays.

The results (mean ± SD) were calculated for each parameter. Following a normality test, a parametric ANOVA with Dunnett's post-test or Pearson correlation was performed on all the experiments. *p<0.05; **<0.01; ***p<0.001. Oleuropein, rutin and rutin+curcumin treatments can decrease OA score in guinea pigs by different mechanisms but only oleuropein can decrease synotivis compared to the control group.

### Example 3

Collagen synthesis by osteoblasts and potentiation of effect by vitamin D were investigated. Primary osteoblasts were isolated from the calvaria of newborn Wistar rats by enzymatic digestion as described by Declercq et al. in 2004. During all experiments, cells were maintained in α-minimal essential medium with 10% heat-inactivated fetal bovine serum and 1% penicillin / streptomycin under conditions of 5% CO2 and 37°C. Cells were seeded on Coll-1-coated 96-well plates at a density of 3500 cells/ well or in 24-well plates at a density of 3×10⁴ cells/well and cultured for 2 days in α-MEM to reach confluence. Cells were then exposed to different conditions: minimal medium (C-), minimal medium containing 50 µg/ml ascorbic acid, 5 mM β-glycerophosphate (C+; optimized medium), and minimal medium supplemented with 1 µM Oleuropein (OLP) and/or 1µM Hydroxytyrosol (HTy) and/or 10⁻⁹ M 1,25 dihydroxivitamin D3 (1,25(OH)2D3). OLP and HTy were dissolved in dimethylsulfoxide (DMSO) and 1,25(OH)2D3 was dissolved in ethanol. The final concentrations of DMSO and ethanol in the medium were 0.1% each. The medium was changed every 2 days.

After 9 days, cellular collagen content was measured using the Sircol Assay from Biocolor. This Sircol Collagen Assay is a quantitative dye-binding method designed for the analysis of collagens extracted from mammalian tissues and cells during in vitro culture. The dye reagent binds specifically to the [Gly-X-Y]n helical structure found in mammalian collagens (Types I-V).

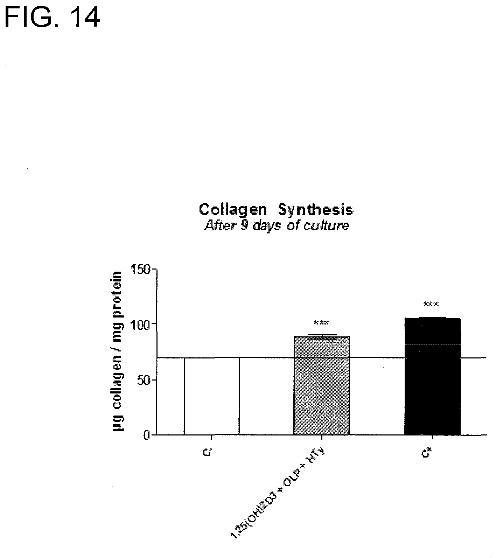

FIG. 14 shows collagen synthesis by osteoblasts after 9 days of culture, measured by micrograms of collagen produced per milligram of total protein in response to stimulation with a combination of 1,25(OH)2D3 OLP and HTy.
1,25(OH)2D3 = 10⁻⁹ M 1,25 dihydroxivitamin D3
OLP = 1 µM oleuropein
HTy = 1µM hydroxytyrosol
C+ = optimized medium used as positive control

Data were expressed as mean ± SEM (3 replicates). Statistics: Kruskal Wallis test followed by Mann Whitney U test (2 tailed) *p<0.05; **p<0.01; ***p<0.001 vs negative control (C-).

As is shown in FIG. 14, the addition of a combination of 1,25(OH)2D3, OLP and HTy stimulates significantly the cellular collagen synthesis of the osteoblasts after 9 days of culture.

### Example 4

Alkaline phosphatase activity of osteoblasts and potentiation of effect by vitamin D were investigated. Primary osteoblasts were isolated from the calvaria of newborn Wistar rats by enzymatic digestion as described by Declercq et al. in 2004. During all experiments, cells were maintained in α-minimal essential medium with 10% heat-inactivated fetal bovine serum and 1% penicillin / streptomycin under conditions of 5% CO2 and 37°C. Cells were seeded on Coll-1-coated 96-well plates at a density of 3500 cells/ well or in 24-well plates at a density of 3×10⁴ cells/well and cultured for 2 days in α-MEM to reach confluence. Cells were then exposed to different conditions: minimal medium (C-), minimal medium containing 50 µg/ml ascorbic acid, 5 mM β-glycerophosphate (C+; optimized medium), and minimal medium supplemented with 1 µM oleuropein (OLP) and/or 1µM hydroxytyrosol (HTy) and/or 10-9 M 1,25 dihydroxivitamin D3 (1,25(OH)2D3). OLP and HTy were dissolved in dimethylsulfoxide (DMSO) and 1,25(OH)2D3 was dissolved in ethanol. The final concentrations of DMSO and ethanol in the medium were 0.1% each. The medium was changed every 2 days.

Enzymatic activity of alkaline phosphatase (ALP) was measured kinetically on Treatment Days 0, 5, 9, 12 and 15. Osteoblasts were rinsed twice with PBS before being frozen on dry ice. The cells were then lysed by freeze-thaw cycle and homogenization into 200 µl of 1 M diethanolamine and 0.24 mM magnesium chloride buffer pH 9.8 (ALP buffer). Cell lysates (10 µl) were added to 200 µl of 6mg/ml solution of p-nitrophenyl phosphate (p-NPP) in ALP buffer. Absorbance was measured at 405 nm and 30 °C, and every 2 min 30 s for 30 min using a VarioSklan Flash microplate reader. ALP activity was expressed as micromoles of p-NPP per hour per milligram of protein. Protein measurement was performed in accordance with the method of Bradford using Quick Start BioRad protein assay.

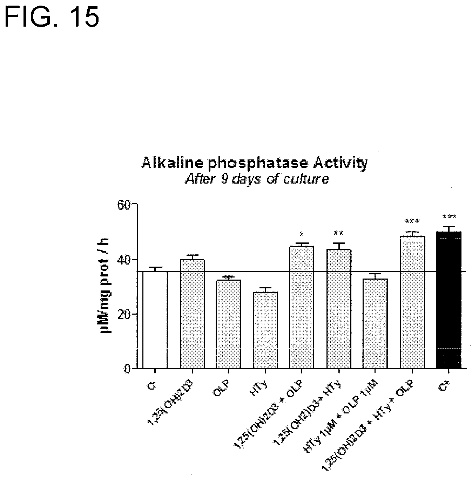

FIG. 15 shows alkaline phosphatase (ALP) activity after 9 days of culture, indicated by micromolar ALP per milligram of protein per hour in response to with a combination of 1,25(OH)2D3, OLP and HTy.
C- = minimal medium use as negative control
1,25(OH)2D3 = 10⁻⁹ M 1,25 dihydroxivitamin D3
OLP = 1 µM oleuropein
HTy = 1µM hydroxytyrosol
C+ = optimized medium used as positive control

Data were expressed as mean ±SEM. Statistics: Kruskal Wallis test followed by Mann Whitney U test (2 tailed) *p<0.05; **p<0.01; ***p<0.001 vs negative control (C-).

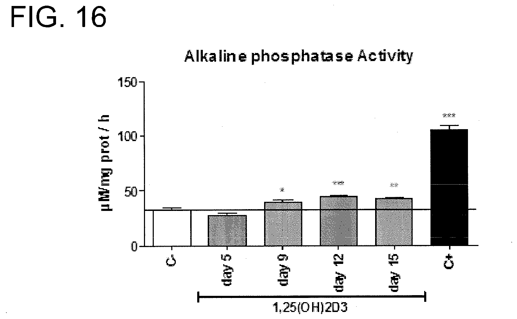

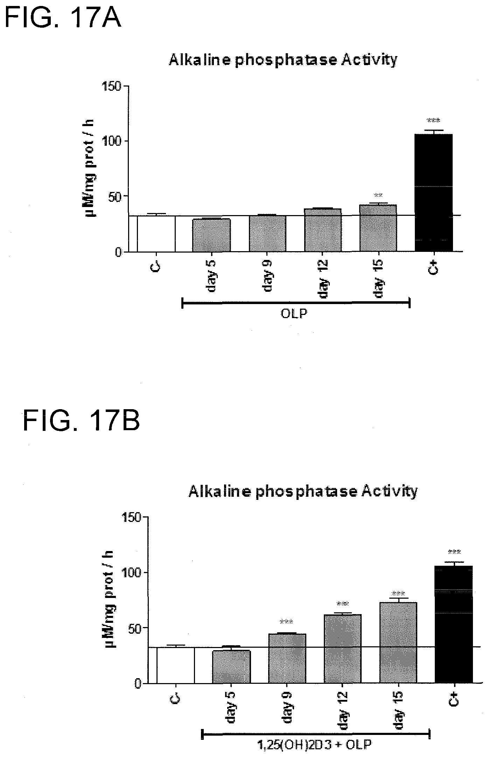

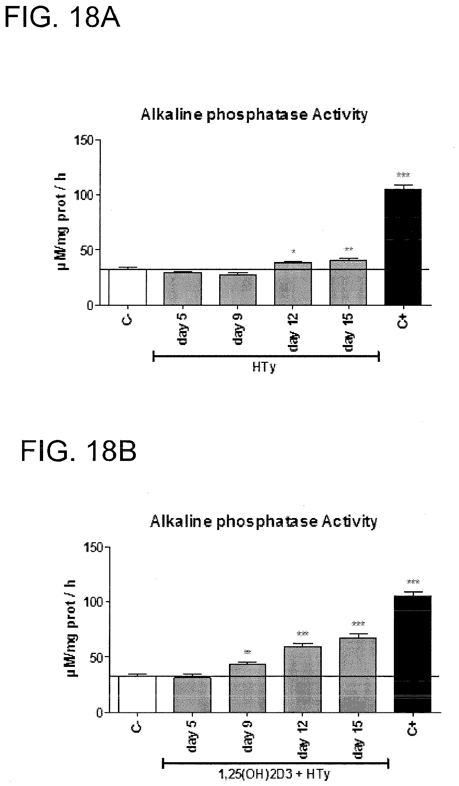

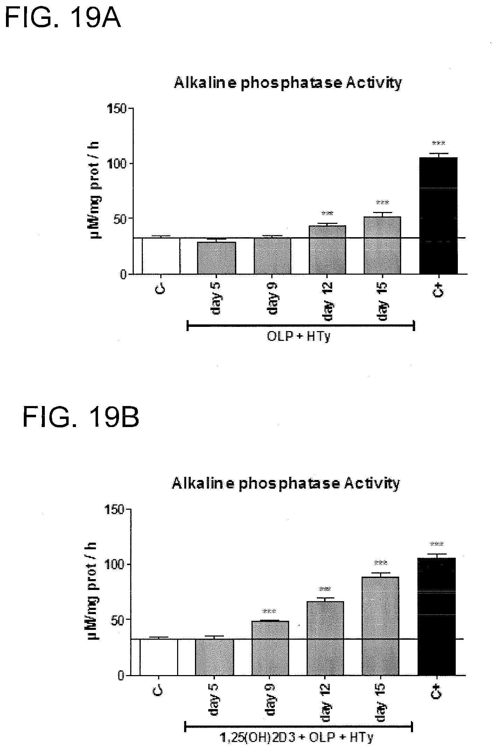

FIG. 15 further demonstrates that the addition of a combination of 1,25(OH)2D3, OLP and HTy stimulates significantly the cellular collagen synthesis of the osteoblasts after 9 days of culture. FIGS. 16-19 show alkaline phosphatase (ALP) activity of osteoblasts cultured in the presence of 1,25(OH)2D3, oleuropein (OLP), hydroxytyrosol (Hyt); separately or in combinations in a time course model.
C- = minimal medium use as negative control
1,25(OH)2D3 = 10⁻⁹ M 1,25 dihydroxivitamin D3
OLP = 1 µM oleuropein
HTy = 1 µM hydroxytyrosol
C+ = optimized medium used as positive control.

Data were expressed as mean ±SEM. Statistics: Kruskal Wallis test followed by Mann Whitney U test (2 tailed) *p<0.05; **p<0.01; ***p<0.001 vs negative control (C-).

As can be seen from the data shown in FIG. 16, the addition of vitamin D3 in itself does induce a small but statistically significant increase in AP activity on day 15, indicative of osteoblast activity and bone formation and/or cartilage anabolism. In the same manner, the addition of either HTy on its own or OLP on its own also results in a small increase on day 15, comparable with the effect of vitamin D3 (see respective figures).

Combining both HTy and OLP does also increase the osteoblast activity (again, looking at day 15), but not as much as combining vitamin D with either HTy or OLP. Still, the only points with statistically significant difference from control are day 12 and day 15. Earlier measurements, at day 5 and day 9, do not differ statistically from negative control.

Thus, the data presented illustrate two effects of addition of vitamin D. Firstly, the addition of vitamin D together with either one or both of the HTy and OLP increases the osteoblast response. This is seen in comparison of for example levels at Day 15. Secondly, the addition of vitamin D together with either one or both of the HTy and OLP elicits osteoblast activity earlier than in the control. The conclusion is that addition of vitamin D leads to an earlier response and a stronger response. It is concluded that addition of vitamin D potentiates the bone formation and/or cartilage anabolism response elicited by OLP and/or HTy.

It should be understood that various changes and modifications to the presently preferred embodiments described herein will be apparent to those skilled in the art. Such changes and modifications can be made without departing from the spirit and scope of the present subject matter and without diminishing its intended advantages. It is therefore intended that such changes and modifications be covered by the appended claims.

## Claims

1. A composition comprising a therapeutically effective amount of a phytonutrient selected from the group consisting of oleuropein, rutin and combinations thereof, for treating synovitis in an individual having synovitis

2. The composition of Claim 1 wherein the synovitis is associated with a condition selected from the group consisting of lupus, gout, arthritis and combinations thereof.

3. The composition of Claim 2 wherein the condition is arthritis that is selected from the group consisting of osteoarthritis, osteochondritis disease, osteoarthrosis and a combination thereof.

4. The composition of Claim 1 wherein the phytonutrient comprises oleuropein.

5. The composition of Claim 1 wherein the composition further comprises an additional bioactive compound selected from the group consisting of curcumin, quercetin, hydroxytyrosol, vitamin D, and combinations thereof.

6. The composition of Claim 1 wherein the phytonutrient is isolated from a plant extract.

7. A composition comprising a therapeutically effective amount of a phytonutrient selected from the group consisting of oleuropein, rutin and combinations thereof, for preventing synovitis in an individual at risk thereof.

8. The composition of Claim 7 wherein the individual is selected from the group consisting of an aging human and an elderly human.

9. The composition of Claim 7 wherein the phytonutrient is isolated from a plant extract.

10. The composition of Claim 7 wherein the composition further comprises an additional bioactive compound selected from the group consisting of curcumin, quercetin, hydroxytyrosol, vitamin D, and combinations thereof.

11. The composition of Claim 7 wherein the composition is administered daily for at least one month.

12. A composition for treating or preventing synovitis comprising a therapeutically effective amount of a phytonutrient selected from the group consisting of oleuropein, rutin and combinations thereof.

13. The composition of Claim 12 wherein the composition is a nutritional composition.

14. The composition of Claim 13 wherein the nutritional composition comprises a component selected from the group consisting of protein, carbohydrate, fat and combinations thereof.

15. A composition comprising a therapeutically effective amount of a phytonutrient selected from the group consisting of oleuropein, rutin and combinations thereof, for treating or preventing articular cartilage degradation subsequent to an individual in need of same.
